# EUROPEAN PATENT APPLICATION

(11) **EP 3 936 605 A1**
(43) Date of publication of application: **12.01.2022**
(21) Application number: 21181783.8
(22) Date of filing: 25.06.2021
(51) Int. Cl.: C12M 3/00, C12M 1/12, C12M 1/26

(54) **CELL TRANSFER METHOD**

(30) Priority: 06.07.2020 JP 2020116655
(71) Applicant: Nihon Kohden Corporation, Shinjuku-ku Tokyo (JP)
(72) Inventor: KUBO, Hirotsugu, Saitama (JP); SHIOYAMA, Takahiro, Saitama (JP); KAGAWA, Yuki, Saitama (JP); HINATA, Yuto, Saitama (JP); SHIMIZU, Tatsuya, Tokyo (JP); SASAKI, Daisuke, Tokyo (JP); HARAGUCHI, Yuji, Tokyo (JP)
(74) Representative: Grünecker Patent- und Rechtsanwälte PartG mbB

(57) **Abstract**

A transfer method includes a centrifugation step for applying centrifugal force in the direction of lamination to a laminate of a cell structure disposed on a temperature-responsive culture dish and a gel placed on the cell structure and a peeling step for maintaining the temperature at the lower critical solution temperature of the temperature-responsive culture dish or lower and peeling off the cell structure containing muscle cells from the temperature-responsive culture dish.

## Description

### TECHNICAL FIELD

The presently disclosed subject matter is a transfer method for transferring a cell structure containing muscle cells to a medium which is capable of cell adhesion.

### BACKGROUND ART

There are needs for transfer of a cell structure containing muscle cells cultured on a temperature-responsive culture dish to a medium which is capable of cell adhesion in a state that is as close as possible to the original state, namely without changing the positional relationships of the individual cells and the shape of the extracellular matrix.

Examples of such a medium include another temperature-responsive culture dish, a hydrogel (a fibrin gel or a collagen gel), a medium having an exposed sensor which can measure the surface potential of cells (a multielectrode array dish or a flexible electrode) and another functional material (silicone or the like). A flexible electrode is flexible and shrinkable and can thus follow any movement of a subject to be measured and continue measurement without peeling off. Silicone has elasticity and oxygen permeability. Using the characteristics, for example, after seeding and culturing epidermal cells on a stretched silicone membrane, the silicone is returned to the original size, and a monolayer of overcrowded cells is reproduced experimentally. By studying peeling of the cells from the monolayer or by culturing the cells on silicone, the oxygen supply from the bottom surface of the culture can be enabled, and the survival of cells with high oxygen demand can be enhanced.

With respect to a specific example for transferring a cell structure containing muscle cells to a medium which is capable of cell adhesion, there is a need for transfer of a pulsating myocardial cell sheet which is cultured on a temperature-responsive culture dish to a fibrin gel.

In this regard, Non-patent Literature 1 and Patent Literature 1 disclose that a mechanism of detecting tension caused by pulsating of myocardial cells with a tension sensor is used for a cardiac toxicity screening test.

In the methods disclosed in Non-patent Literature 1 and Patent Literature 1, a fibrin gel sheet is first placed on the cells (before peeling) cultured on a temperature-responsive culture dish, and the gel sheet is pressed onto the cells by placing a weight from above. When the cells are then cultured for 60 minutes in an environment at 20°C while the gel sheet is pressed onto the cells, the cells peel off from the temperature-responsive culture dish, and the cells can be transferred to the surface of the fibrin gel sheet.

The present inventors have found problems of the transfer methods disclosed in Non-patent Literature 1 and Patent Literature 1, that is, when the operations are not carried out carefully in the steps of placing the weight on the fibrin gel and replacing after the culture at 20°C for 60 minutes, the cells are not transferred evenly to the surface of the fibrin gel sheet, and the cells are partially peeled off or die.

### CITATION LIST

### PATENT LITERATURE

[Patent Literature 1] JP2019-76046A

### NON-PATENT LITERATURE

[Non-patent Literature 1] Sasaki et al. (2018) PLoSONE 13: e0198026

### SUMMARY

The presently disclosed subject matter has been made to solve the problems and aims to provide a transfer method which can easily and evenly transfer a cell structure containing muscle cells to a medium which is capable of cell adhesion.

As a result of intensive investigation to solve the problems, the inventors have found that the problems can be solved by a transfer method for transferring a cell structure containing muscle cells to a medium which is capable of cell adhesion which includes a centrifugation step for applying centrifugal force in the direction of lamination to a laminate of the cell structure disposed on a temperature-responsive culture dish and the member for transfer placed on the cell structure and a peeling step for maintaining the temperature at the lower critical solution temperature of the temperature-responsive culture dish or lower and peeling off the cell structure containing muscle cells from the temperature-responsive culture dish, and the presently disclosed subject matter has been thus completed.

According to the transfer method described above, a cell structure containing muscle cells can be easily and evenly transferred to a medium which is capable of cell adhesion.

### BRIEF DESCRIPTION OF DRAWINGS

[FIG. 1] A schematic perspective view of a tension measuring device according to an embodiment of the presently disclosed subject matter.
[FIG. 2] A schematic view of assembled gel adaptor holders.
[FIG. 3] A schematic view of a first gel adaptor holder.
[FIG. 4] A schematic view of a second gel adaptor holder.
[FIG. 5] FIGS. 5A to 5D are figures for explaining the method for transferring a cell structure to a gel formed in assembled gel adaptor holders.
[FIG. 6] FIG. 6A is a schematic view of a gel formed in assembled gel adaptor holders, and FIG. 6B is a section view along the line 6B-6B in FIG. 6A and illustrates how a gel is placed on a cell structure disposed on a temperature-responsive culture dish.
[FIG. 7] A plan view of assembled gel adaptor holders having a gel placed on a cell structure disposed on a temperature-responsive culture dish.
[FIG. 8] FIGS. 8A to 8B are schematic views of the centrifugation step of the transfer method according to the embodiment.
[FIG. 9] FIGS. 9A to 9B are figures of a tension measuring device according to a modified example corresponding to FIGS. 6A to 6B
[FIG. 10] FIGS. 10A to 10C are figures of a tension measuring device according to a modified example corresponding to FIG. 7.

### DESCRIPTION OF EMBODIMENTS

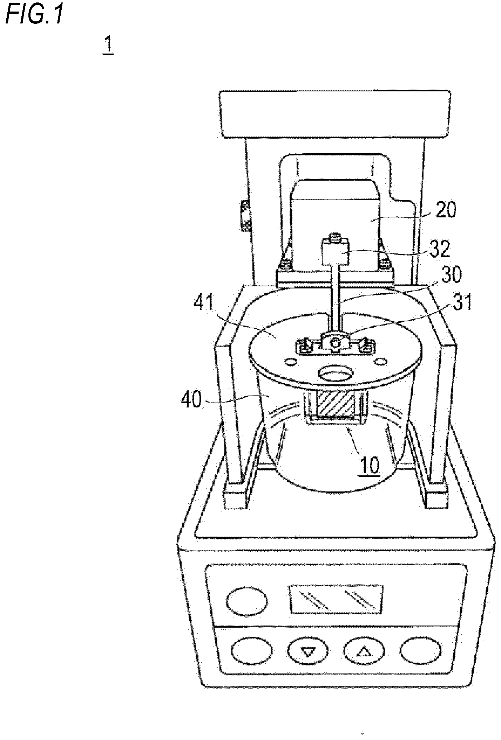

An embodiment of the presently disclosed subject matter is explained below referring to FIG. 1 to FIG. 8B. In the explanation of the drawings, a same reference sign is given to same elements, and overlapping explanations are omitted. The ratios of sizes in the drawings are exaggerated for the explanation and are sometimes different from the actual ratios.

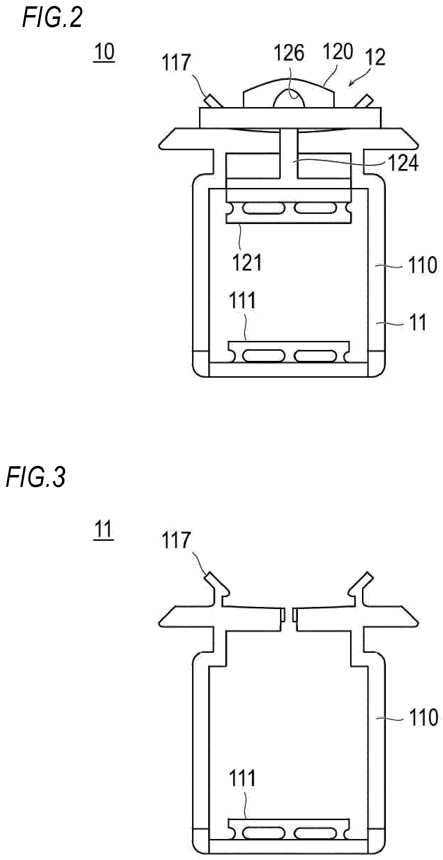

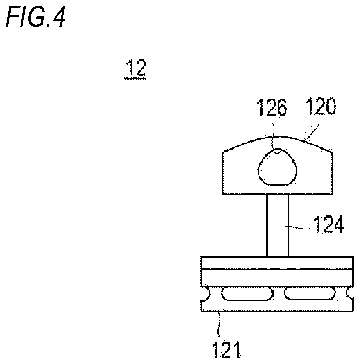

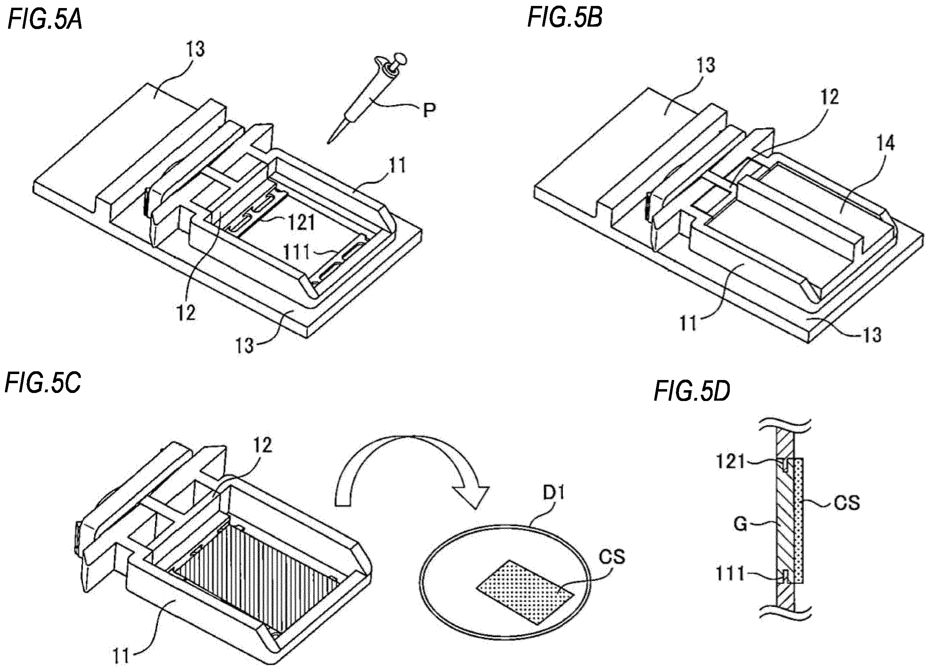

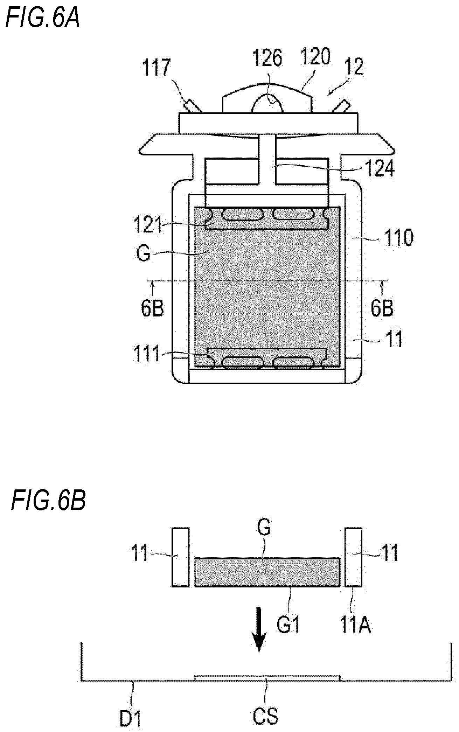

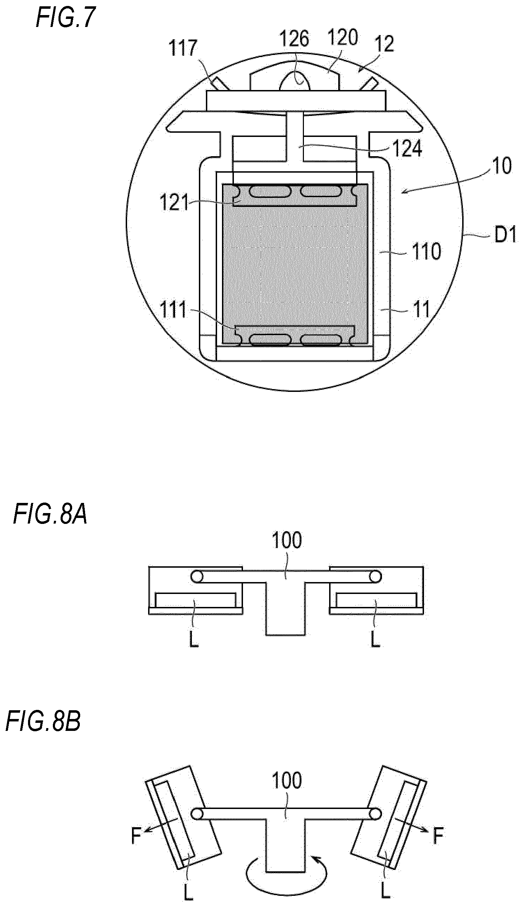

FIG. 1 is a schematic perspective view of a tension measuring device 1 according to an embodiment of the presently disclosed subject matter. FIG. 2 is a schematic view of assembled gel adaptor holders 10. FIG. 3 is a schematic view of a first gel adaptor holder 11. FIG. 4 is a schematic view of a second gel adaptor holder 12. FIGS. 5A to 5D are figures for explaining the method for transferring a cell structure CS to a gel G formed in the assembled gel adaptor holders 10. FIGS. 6A and 6B are schematic views illustrating how the gel G is placed on the cell structure CS disposed on a temperature-responsive culture dish D1. FIG. 7 is a plan view of the assembled gel adaptor holders 10 having the gel G placed on the cell structure CS disposed on the temperature-responsive culture dish D1. FIGS. 8A and 8B are schematic views of the centrifugation step of the transfer method according to the embodiment.

Before explaining the configuration of a tension measuring device 1 according to the embodiment, a cell structure CS containing muscle cells, which is the subject to be measured by the tension measuring device 1, is explained.

### <Cell structure containing muscle cells>

In the specification, "cell structure containing muscle cells" means living body tissue (for example, myocardial tissue, skeletal muscle tissue, or smooth muscle tissue) containing muscle cells collected from the living body, or a structure containing muscle cells. The cell structure containing muscle cells that can be applied to the presently disclosed subject matter may be living body tissue itself collected from the living body, myocardial tissue or the like using human iPS cell-derived myocardial cells for example, or living body tissue that is obtained by processing living body tissue collected from the living body. Alternatively, the cell structure containing muscle cells that can be applied to the presently disclosed subject matter may be a cell structure that is formed by mixing a suspension containing muscle cells with a gel solution or a gelling agent, or a cell sheet. Alternatively, the cell structure containing muscle cells that can be applied to the presently disclosed subject matter may be a structure that is formed by directly seeding and culturing a cell group containing muscle cells on a gel.

In the embodiment, the cell structure containing muscle cells that can be applied to the presently disclosed subject matter may be at least one selected from a group consisting of a sheet-like shape, a rod-like shape, and a string-like shape, and preferably a sheet-like cell structure.

In the specification, "sheet-like cell structure" means a film-like cell structure having an average thickness of, for example, about 10 µm (e.g., the thickness of one cell) or more and about 2 mm or less, and having a length that enables the structure to be applied between first and second gel holding portions which will be described later. The width of "sheet-like cell structure" is requested to have a value that enables the structure to be applied to the first and second gel holding portions, and not particularly limited. One "sheet-like cell structure" may be applied to the tension measuring device, or a plurality of "sheet-like cell structures" may be applied to the tension measuring device. The plurality of "sheet-like cell structures" may be applied in parallel between the first and second gel holding portions, or applied in a stacked manner.

In the specification, "rod-like cell structure" means a cell structure having an average diameter of, for example, about 100 µm or more and about 5 mm or less, and having a length that enables the structure to be applied between the first and second gel holding portions which will be described later. One "rod-like cell structure" may be applied to the tension measuring device, or a plurality of "rod-like cell structures" may be applied to the tension measuring device. The plurality of "rod-like cell structures" may be applied in parallel between the first and second gel holding portions, or applied in a bundled manner.

In the specification, "string-like cell structure" means a three-dimensional cell structure having an average diameter of, for example, about 10 µm or more and less than about 100 µm, and having a length that enables the structure to be applied between the first and second gel holding portions which will be described later. One "string-like cell structure" may be applied to the tension measuring device, or a plurality of "string-like cell structures" may be applied to the tension measuring device. The plurality of "string-like cell structures" may be applied in parallel between the first and second gel holding portions, or applied in a bundled manner.

Although, for the sake of convenience, "rod-like cell structure" and "string-like cell structure" are separately described depending on the diameter, both the terms mean a long thin shape cell structure, and may be used interchangeably.

For example, "rod-like cell structure" and "string-like cell structure" that can be applied to the presently disclosed subject matter may be produced by forming (for example, winding, twisting, constricting, or cutting) a sheet-like cell structure into a rod-like shape or a string-like shape, or by pouring a suspension containing cells and an arbitrary gel into a mold having a rod-like or string-like recess (for example, see Zhao Y, Cell. 2019 Feb 7; 176(4): 913-927).

In the specification, "cell structure containing muscle cells" means a cell structure that, with respect to the number of cells contained in the cell structure, contains at least 1% or more of muscle cells. For example, muscle cells are contained at 15% or more, 20% or more, 25% or more, 30% or more, 35% or more, 40% or more, 45% or more, 50% or more, 55% or more, 60% or more, 65% or more, 70% or more, 75% or more, 80% or more, 85% or more, 90% or more, 95% or more, or 99% or more. In the specification, "muscle cells" means cells that form muscle tissue of an animal body, and that have contractility. Examples of such cells are myocardial cells, skeletal muscle cells, and smooth muscle cells. In the embodiment, "muscle cells" that is used in the presently disclosed subject matter is at least one selected from a group consisting of myocardial cells, skeletal muscle cells, and smooth muscle cells. The muscle cells that can be used in the presently disclosed subject matter are requested to be animal-derived. For example, muscle cells of a mammal, a bird, an amphibian, a reptile, or a fish can be used. Mammal-derived muscle cells are preferably used. For example, muscle cells derived from a mammal such as a mouse, a rat, a human, a monkey, a pig, a dog, a sheep, a cat, or a goat may be used.

Muscle cells that can be used in the presently disclosed subject matter may be primary cells that are collected from living body tissue, established cells, or cells that are differentiated and induced from pluripotent stem cells or tissue stem cells.

In the specification, "pluripotent stem cells" is intended to generally mean stem cells having the ability to differentiate into cells of any kind of tissue (pluripotent differentiation). Although not limited, the pluripotent stem cells include embryonic stem cells (ES cells), embryonic carcinoma cells (EC cells), trophoblast stem cells (TS cells), epiblast stem cells (EpiS cells), embryonic germ cells (EG cells), multipotent germline stem cells (mGS cells), induced pluripotent stem cells (iPS cells), Muse cells, or the like. Preferably, ES cells or iPS cells are used. Although, as the pluripotent stem cells, arbitrary known pluripotent stem cells can be used, pluripotent stem cells disclosed in, for example, WO/2009/123349 (PCT/JP2009/057041) may be used.

Muscle cells that are useful in the presently disclosed subject matter may be cells that are differentiated and induced from pluripotent stem cells. As a method of differentiating from pluripotent stem cells to muscle cells, a known method can be used (for example, see Matsuura K., et al. Creation of human cardiac cell sheets using pluripotent stem cells. Biochem. Biophys. Res. Commun. 2012 Aug 24; 425(2): 321-327).

The cell structure containing muscle cells may contain cells other than muscle cells. For example, cardiac myoblasts, myoblast cells, mesenchymal stem cells, vascular endothelial cells, vascular endothelial precursor cells, fibroblasts, and the like may be contained.

The cell structure CS containing muscle cells has been explained above. Next, the configuration of the tension measuring device 1 according to the embodiment is explained.

As illustrated in FIG. 1, the tension measuring device 1 can include assembled gel adaptor holders 10 in which a gel G and a cell structure CS are held, a tension detecting unit 20 which detects the tension caused by pulsation of the cell structure CS, a connecting member 30 which connects the assembled gel adaptor holders 10 and the tension detecting unit 20 and a container 40 in which the cell structure CS and the assembled gel adaptor holders 10 are disposed.

As illustrated in FIG. 2 to FIG. 5D, the gel G is formed in the assembled gel adaptor holders 10.

As the gel that can be used in the presently disclosed subject matter, a gel can be used as far as (1) a cell structure containing muscle cells can be adhered to the gel, (2) the gel has a strength that can maintain the sheet shape, and (3) the gel does not give an adverse effect on the growth of cells, the expression of a function, i.e., as far as the gel is biocompatible. An example of the gel that can be used in the presently disclosed subject matter is a hydrogel. Examples of the hydrogel that can be applied to the presently disclosed subject matter is a hydrogel in which a water-soluble, hydrophilic, or water-absorbing synthetic polymer such as polyacrylamide, polyacrylic acid, polyhydroxyethylmethacrylate, polyvinyl alcohol, polylactate, and polyglycolic acid, polysaccharide, protein, or nucleic acid is chemically linked. Examples of polysaccharide are glycosaminoglycan such as hyaluronic acid or chondroitin sulfuric acid, starch, glycogen, agarose, pectin, cellulose, and the like. Examples of protein are collagen, gelatin that is a hydrolysate of collagen, proteoglycan, fibronectin, vitronectin, laminin, entactin, tenascin, thrombospondin, a von Willebrand factor, osteopontin, fibrinogen (for example, a fibrin gel in which fibrinogen and thrombin are reacted with each other), and the like. Such a hydrogel may be used after applying a cross-linking treatment to the hydrogel by using a known method, to enhance the strength. Preferably, the gel that can be applied to the presently disclosed subject matter is a fibrin gel. In the embodiment of the presently disclosed subject matter, the gel may be obtained by previously mixing the gel with cells.

An example of the configuration of the assembled gel adaptor holders 10 is explained below, but the configuration of the assembled gel adaptor holders 10 is not limited to the following configuration as long as the gel G and the cell structure CS can be held.

Those described in JP2019-036677 can be used as the assembled gel adaptor holders 10. Therefore, the details of the assembled gel adaptor holders 10 are not explained here, and only the summary of the configuration is explained.

As illustrated in FIG. 2 to FIG. 4, the assembled gel adaptor holders 10 can include a first gel adaptor holder 11 and a second gel adaptor holder 12.

The first gel adaptor holder 11 and the second gel adaptor holder 12 are assembled as illustrated in FIG. 2 and thus constitute the assembled gel adaptor holders 10.

As illustrated in FIG. 2 and FIG. 3, the first gel adaptor holder 11 can include a frame member 110, a first gel holding portion 111 for fixing one end of the gel G and a pair of claw portions 117 fixed on a cover 41 of the container 40.

When the frame member 110 is disposed, the assembled gel adaptor holders 10 can be attached to the cover 41 (described later) of the container 40 while the shapes of the gel G and the cell structure CS are maintained.

The material constituting the first gel adaptor holder 11 is not particularly limited, but examples include polyethylene, polypropylene, polycarbonate, polystyrene, polyvinyl chloride, nylon, polyurethane, polyurea, polylactate, polyglycolic acid, polyvinyl alcohol, polyvinyl acetate, poly(meth)acrylic acid, poly(meth)acrylic acid derivatives, polyacrylonitrile, poly(meth)acrylamide, poly(meth)acrylamide derivatives, polysulfone, cellulose, cellulose derivatives, polysilicone, metals and the like.

As illustrated in FIG. 2 and FIG. 4, the second gel adaptor holder 12 can include a connecting portion 120 to which the connecting member 30 is connected, a second gel holding portion 121 for fixing the other end of the gel G and a coupling portion 124 which connects the connecting portion 120 and the second gel holding portion 121.

In the connecting portion 120, a connecting port 126 for connecting to the connecting member 30 is disposed.

As the material constituting the second gel adaptor holder 12, the same material as that constituting the first gel adaptor holder 11 can be used.

The method for forming the gel G in the first gel adaptor holder 11 and the second gel adaptor holder 12 and the method for transferring the cell structure CS to the gel G (corresponding to the medium which is capable of cell adhesion) formed in the assembled gel adaptor holders 10 are explained below referring to FIG. 5A to FIG. 8B.

First, the first gel adaptor holder 11 and the second gel adaptor holder 12 are set on a substrate 13, and a gelling agent (for example, a mixture of fibrinogen (SIGMA, derived from bovine blood plasma, Type I-S), thrombin (SIGMA, derived from bovine blood plasma, T4648), a CaCl₂ solution (8 mM), and Factor XIII (CSL Behring, intravenous Fibrogamin P)) before curing is poured by using a pipette P (FIG. 5A).

Next, the first gel holding portion 111 and the second gel holding portion 121 are covered with a gel forming cover 14 (FIG. 5B).

After the gel is cured, the gel forming cover 14 and the substrate 13 are removed from the first gel adaptor holder 11 and the second gel adaptor holder 12 (FIG. 5C). Here, as illustrated in FIG. 6B, the gel G is formed in a manner that the bottom surface G1 of the gel G is on an approximately same plane as that of the bottom surface 11A of the first gel adaptor holder 11.

Separately from the above, a cell group containing muscle cells is seeded onto a temperature-responsive culture dish D1 (for example, UpCell (registered trademark), (CellSeed Inc., Tokyo, Japan)), and cells are previously cultured at 37°C until becoming confluent (FIG. 5C).

The assembled gel adaptor holders 10 having the gel obtained above are placed on the cell structure CS on the temperature-responsive culture dish D1 (FIG. 5C). Here, as illustrated in FIG. 7, the size of the temperature-responsive culture dish D1 is preferably a size in which the assembled gel adaptor holders 10 just fit. According to the configuration, unintended movement of the assembled gel adaptor holders 10 can be favorably prevented during the centrifugation step described later.

As illustrated in FIG. 8B, centrifugal force F is applied in the direction of lamination to the laminate L of the cell structure CS disposed on the temperature-responsive culture dish D1 and the gel G placed on the cell structure CS (a centrifugation step). The centrifugation step is conducted using a known plate centrifuge 100. The period of the centrifugation step is not particularly limited but is, for example, five minutes.

In the centrifugation step, the direction of lamination of the laminate L is in the vertical direction in the beginning of rotation as illustrated in FIG. 8A, and when the rotation speed is increased, the laminate L rises in a manner that the direction of lamination of the laminate L becomes an approximately horizontal direction as illustrated in FIG. 8B. As a result, the gel G is physically pressed onto the cell structure CS by the centrifugal force F.

The laminate L is then placed in a thermostatic chamber and cultured in an environment of 5% CO₂ at 37°C. Through the step, the cell structure CS biologically actively adheres to the gel G (an adhesion step). The period of the adhesion step is not particularly limited but is, for example, 60 minutes.

The laminate L is then cultured in a thermostatic chamber in an environment of 5% CO₂ at 20°C. Through the step, the cell structure CS is peeled off from the temperature-responsive culture dish D1 (a peeling step). The period of the peeling step is not particularly limited but is, for example, 90 minutes.

When a cell structure CS was transferred to a gel G by the transfer method explained above, the cell structure CS could be transferred with certainty of 100%. In this regard, however, the success rate of transfer was 21% with the method using a weight explained in the Background Art.

Hereinafter, the configuration of the tension measuring device 1 is further explained.

The tension detecting unit 20 measures the tension caused by pulsation of the cell structure CS containing muscle cells. As illustrated in FIG. 1, the tension detecting unit 20 is connected to the assembled gel adaptor holders 10 through the connecting member 30. When the cell structure CS containing muscle cells contracts, the second gel adaptor holder 12 is pulled downward in the vertical direction, and the load is detected by the tension detecting unit 20 through the connecting member 30. For example, a known load cell can be used as the tension detecting unit 20.

As illustrated in FIG. 1, the connecting member 30 connects the assembled gel adaptor holders 10 and the tension detecting unit 20. The connecting member 30 can include a hook 31 and a connecting portion 32.

The hook 31 is inserted into the connecting port 126 of the second gel adaptor holder 12. The connecting portion 32 is connected to the tension detecting unit 20.

The connecting member 30 is not limited to the above configuration as long as the second gel adaptor holder 12 and the tension detecting unit 20 can be connected.

In the container 40, the assembled gel adaptor holders 10 having the gel G and the cell structure CS are contained. The cover 41 is attached to the container 40.

The pair of claw portions 117 of the first gel adaptor holder 11 fit into the cover 41.

A medical agent and a medium are injected into the container 40. A heater (not illustrated) is disposed below the container 40.

As explained above, the transfer method according to the embodiment is a transfer method for transferring a cell structure CS containing muscle cells to a gel G which is capable of cell adhesion. The transfer method can include a centrifugation step for applying centrifugal force F in the direction of lamination to a laminate L of the cell structure CS disposed on a temperature-responsive culture dish D1 and the gel G placed on the cell structure CS and a peeling step for maintaining the temperature at the lower critical solution temperature of the temperature-responsive culture dish D1 (at 20°C in the embodiment) or lower and peeling off the cell structure CS containing muscle cells from the temperature-responsive culture dish D1. According to the transfer method, the gel G is physically pressed onto the cell structure CS by the centrifugal force F in the centrifugation step, and thus the cell structure CS containing muscle cells can be easily and evenly transferred to the gel G.

The transfer method can also include an adhesion step for maintaining the temperature at the lower critical solution temperature of the temperature-responsive culture dish D1 or higher and causing biologically active adhesion of the cell structure CS containing muscle cells to the gel G between the centrifugation step and the peeling step. According to the transfer method, the cell structure CS containing muscle cells can be more preferably transferred to the gel G.

The presently disclosed subject matter is not limited to the above embodiment and can be variously modified within the scope of the claims.

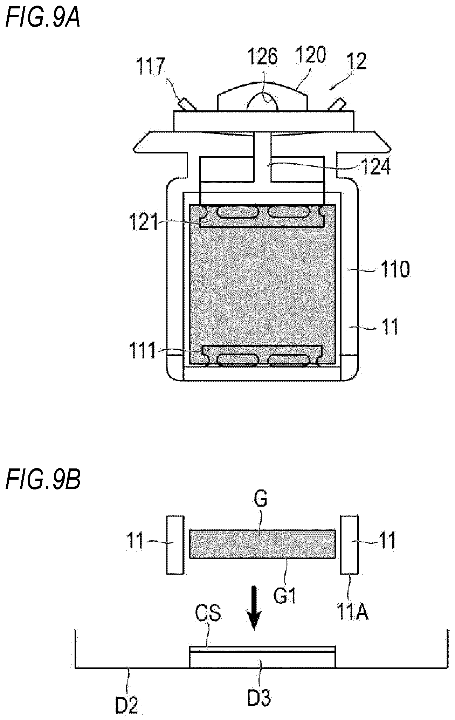

For example, in the above embodiment, as illustrated in FIG. 6B, the gel G is formed in a manner that the bottom surface 11A of the first gel adaptor holder 11 is on an approximately same plane as that of the bottom surface G1 of the gel G. However, as illustrated in FIG. 9B, the gel G may be formed in a manner that the bottom surface G1 of the gel G is higher than the bottom surface 11A of the first gel adaptor holder 11. Here, as illustrated in FIG. 9B, a convex part D3 having a height that is approximately the same as the difference in height between the bottom surface 11A of the first gel adaptor holder 11 and the bottom surface G1 of the gel G is formed on a temperature-responsive culture dish D2, and the cell structure CS is cultured on the convex part D3. When the gel G is formed in this manner, in which the bottom surface G1 of the gel G is higher than the bottom surface 11A of the first gel adaptor holder 11, the sliding of the gel G can be preferably prevented even when the first gel adaptor holder 11 and the second gel adaptor holder 12 are unintendedly moved in the horizontal direction, and the gel G can be prevented from being polluted.

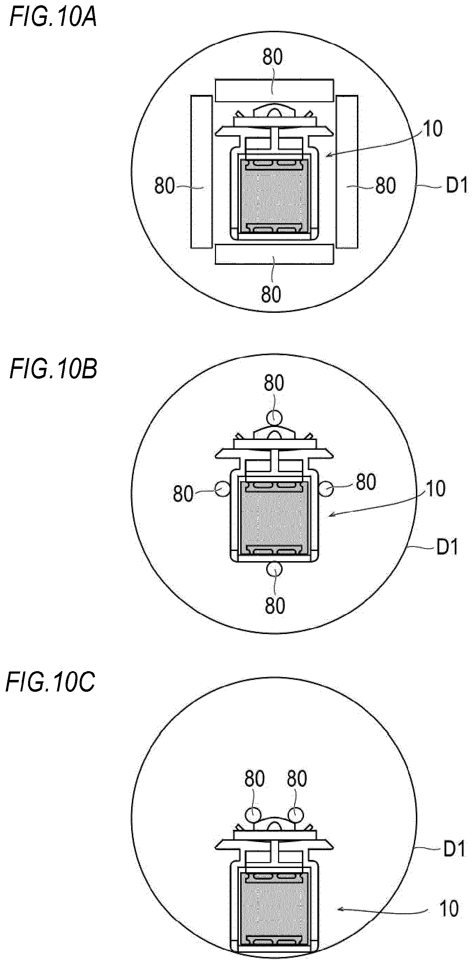

In the above embodiment, as illustrated in FIG. 7, the size of the temperature-responsive culture dish D1 is a size in which the assembled gel adaptor holders 10 just fit. However, as illustrated in FIG. 10A, FIG. 10B and FIG. 10C, the temperature-responsive culture dish D1 may be configured in a size larger than the assembled gel adaptor holders 10. Here, to prevent unintended movement of the assembled gel adaptor holders 10 in the temperature-responsive culture dish D1, controlling members 80 are preferably disposed around the assembled gel adaptor holders 10. The controlling members 80 can be configured as illustrated in FIG. 10A to FIG. 10C. The material constituting the controlling members 80 is not particularly limited, but, for example, silicone can be used. The controlling members 80 are disposed around the assembled gel adaptor holders 10 and may be separate members from the temperature-responsive culture dish D1, and the controlling members 80 may be formed in the temperature-responsive culture dish D1.

In the above embodiment, the transfer method includes the adhesion step for causing biologically active adhesion of the cell structure CS containing muscle cells to the gel G between the centrifugation step and the peeling step. However, the transfer method does not have to include the adhesion step.

In the above embodiment, the adhesion step is conducted in an environment of 5% CO₂ at 37°C for 60 minutes, but the environment and the period are not limited to those described above as long as the cell structure CS containing muscle cells biologically actively adheres to the gel G.

In the above embodiment, the adhesion step is conducted after the centrifugation step. However, by conducting the centrifugation step in a thermostatic chamber, the adhesion step and the centrifugation step may be conducted simultaneously. According to the transfer method, the time required for the transfer can be shortened.

In the above embodiment, the centrifugation step is conducted for five minutes. However, the centrifugation step is not limited to five minutes as long as the effects of the presently disclosed subject matter are exhibited.

In the above embodiment, the peeling step is conducted in an environment of 5% CO₂ at 20°C for 90 minutes, but the environment and the period are not limited to those described above as long as the cell structure CS containing muscle cells can be peeled off from the temperature-responsive culture dish D1.

In the above embodiment, the medium is a hydrogel sheet, but the medium is not limited thereto and may be another temperature-responsive culture dish, a medium having an exposed sensor which can measure the surface potential of cells (a multielectrode array dish or a flexible electrode), another functional material (silicone or the like) or the like.

## Claims

1. Atransfer method for transferring a cell structure containing muscle cells to a medium which is capable of cell adhesion, comprising:
a centrifugation step for applying centrifugal force in a direction of lamination to a laminate of the cell structure disposed on a temperature-responsive culture dish and the medium placed on the cell structure, and
a peeling step for maintaining a temperature at a lower critical solution temperature of the temperature-responsive culture dish or lower and peeling off the cell structure from the temperature-responsive culture dish.

2. The transfer method according to claim 1, further including an adhesion step for maintaining the temperature at the lower critical solution temperature of the temperature-responsive culture dish or higher and causing biologically active adhesion of the cell structure to the medium between the centrifugation step and the peeling step.

3. The transfer method according to claim 2, wherein the adhesion step is conducted in a CO₂ environment at 20°C or higher and 38°C or lower for five minutes or longer.

4. The transfer method according to claim 2 or 3, wherein the adhesion step and the centrifugation step are conducted simultaneously.

5. The transfer method according to any one of claims 1 to 4,
wherein the centrifugation step is conducted for one minute or longer, and
wherein the peeling step is conducted in a CO₂ environment at 20°C for 10 minutes or longer.

6. The transfer method according to any one of claims 1 to 5, wherein the medium is a hydrogel sheet.
